# EUROPEAN PATENT APPLICATION

(11) **EP 4 607 318 A1**
(43) Date of publication of application: **27.08.2025**
(21) Application number: 25155460.6
(22) Date of filing: 03.02.2025
(51) Int. Cl.: G06F 1/20, A61B 5/01, G06F 9/50

(54) **APPARATUS, METHOD, AND SYSTEM FOR MULTI-DEVICE WEARABILITY-AWARE HEAT ORCHESTRATION**

(30) Priority: 21.02.2024 GB 202402466
(71) Applicant: Nokia Technologies Oy, 02610 Espoo (FI)
(72) Inventor: GONG, Taesik, Cambridge (GB); MIN, Chulhong, Cambridge (GB); JANG, Si Young, Cambridge (GB)
(74) Representative: Nokia EPO representatives

(57) **Abstract**

An approach is provided for multiple-device wearability-aware heat orchestration. The approach involves, for example, determining a predicted skin temperature at a skin contact point of a wearable device. The approach also involves determining a wear status of the wearable device. The approach further involves determining a wearable temperature threshold of the wearable device based on the wear status. The approach further involves delegating at least one portion of a computational workload of the wearable device to at least one other device based on determining that the predicted skin temperature is greater than the wearable temperature threshold.

## Description

### FIELD

The disclosed subject matter generally relates to task orchestration across multiple wearable and/or other user devices for thermal management.

### BACKGROUND

Mobile devices (e.g., wearable devices, smartphones, etc.) increasingly perform processing-heavy and complex on-device tasks. However, in the case of wearable devices that come in direct contact with or in close proximity to a user's skin, these on-device tasks and related activities can generate a significant amount of heat that can lead to user discomfort or even serious health threats (e.g., thermal pain, skin burns, skin aging, etc.). Accordingly, device manufacturers and service providers face significant technical challenges to provide high quality of service for on-device tasks while also minimizing adverse skin effects.

### SOME EXAMPLE EMBODIMENTS

Therefore, there is a need for multi-device wearability-aware heat orchestration.

According to one example embodiment, an apparatus comprises means for determining a predicted skin temperature at a skin contact point of a wearable device. The apparatus also comprises means for determining a wear status of the wearable device. The apparatus further comprises means for determining a wearable temperature threshold of the wearable device based on the wear status. The apparatus further comprises means for delegating at least one portion of a computational workload of the wearable device to at least one other device based on determining that the predicted skin temperature is greater than the wearable temperature threshold.

According to another embodiment, an apparatus comprises at least one processor, and at least one memory storing instructions that, when executed by the at least one processor, cause the apparatus at least to determine a predicted skin temperature at a skin contact point of a wearable device. The apparatus is also caused to determine a wear status of the wearable device. The apparatus is further caused to determine a wearable temperature threshold of the wearable device based on the wear status. The apparatus is further caused to delegate at least one portion of a computational workload of the wearable device to at least one other device based on determining that the predicted skin temperature is greater than the wearable temperature threshold.

According to another embodiment, a method comprises determining a predicted skin temperature at a skin contact point of a wearable device. The method also comprises determining a wear status of the wearable device. The method further comprises determining a wearable temperature threshold of the wearable device based on the wear status. The method further comprises delegating at least one portion of a computational workload of the wearable device to at least one other device based on determining that the predicted skin temperature is greater than the wearable temperature threshold.

According to another embodiment, a computer program comprising instructions which, when executed by an apparatus, cause the apparatus to determine a predicted skin temperature at a skin contact point of a wearable device. The apparatus is also caused to determine a wear status of the wearable device. The apparatus is further caused to determine a wearable temperature threshold of the wearable device based on the wear status. The apparatus is further caused to delegate at least one portion of a computational workload of the wearable device to at least one other device based on determining that the predicted skin temperature is greater than the wearable temperature threshold.

According to another embodiment, a computer program comprises instructions for causing an apparatus to determine a predicted skin temperature at a skin contact point of a wearable device. The apparatus is also caused to determine a wear status of the wearable device. The apparatus is further caused to determine a wearable temperature threshold of the wearable device based on the wear status. The apparatus is further caused to delegate at least one portion of a computational workload of the wearable device to at least one other device based on determining that the predicted skin temperature is greater than the wearable temperature threshold.

According to another embodiment, a non-transitory computer-readable storage medium comprising program instructions that, when executed by an apparatus, cause the apparatus to determine a predicted skin temperature at a skin contact point of a wearable device. The apparatus is also caused to determine a wear status of the wearable device. The apparatus is further caused to determine a wearable temperature threshold of the wearable device based on the wear status. The apparatus is further caused to delegate at least one portion of a computational workload of the wearable device to at least one other device based on determining that the predicted skin temperature is greater than the wearable temperature threshold.

According to one example embodiment, an apparatus comprises modeling circuitry configured to perform determining a predicted skin temperature at a skin contact point of a wearable device. The apparatus also comprises detection circuitry configured to perform determining a wear status of the wearable device. The modeling circuitry is further configured to perform determining a wearable temperature threshold of the wearable device based on the wear status. The apparatus further comprises task orchestration circuitry for delegating at least one portion of a computational workload of the wearable device to at least one other device based on determining that the predicted skin temperature is greater than the wearable temperature threshold.

According to one example embodiment, a system comprises one or more devices comprising one or more of a wearable device, a cloud server device, an edge device, an internet of things (IoT) device, a user equipment device, or any combination thereof. The one or more devices are configured to determine a predicted skin temperature at a skin contact point of a wearable device. The one or more devices are also configured to determine a wear status of the wearable device. The one or more devices are further configured to determine a wearable temperature threshold of the wearable device based on the wear status. The one or more devices are further configured to delegate at least one portion of a computational workload of the wearable device to at least one other device based on determining that the predicted skin temperature is greater than the wearable temperature threshold.

According to a further embodiment, a device comprises at least one processor; and at least one memory comprising a computer program code for one or more programs, the at least one memory and the computer program code configured to, with the at least one processor, cause the device to determine a predicted skin temperature at a skin contact point of a wearable device. The device is also caused to determine a wear status of the wearable device. The device is further caused to determine a wearable temperature threshold of the wearable device based on the wear status. The device is further caused to delegate at least one portion of a computational workload of the wearable device to at least one other device based on determining that the predicted skin temperature is greater than the wearable temperature threshold.

In addition, for various example embodiments of the invention, the following is applicable: a method comprising facilitating a processing of and/or processing (1) data and/or (2) information and/or (3) at least one signal, the (1) data and/or (2) information and/or (3) at least one signal based, at least in part, on (or derived at least in part from) any one or any combination of methods (or processes) disclosed in this application as relevant to any embodiment of the invention.

For various example embodiments of the invention, the following is also applicable: a method comprising facilitating access to at least one interface configured to allow access to at least one service, the at least one service configured to perform any one or any combination of network or service provider methods (or processes) disclosed in this application.

For various example embodiments of the invention, the following is also applicable: a method comprising facilitating creating and/or facilitating modifying (1) at least one device user interface element and/or (2) at least one device user interface functionality, the (1) at least one device user interface element and/or (2) at least one device user interface functionality based, at least in part, on data and/or information resulting from one or any combination of methods or processes disclosed in this application as relevant to any embodiment of the invention, and/or at least one signal resulting from one or any combination of methods (or processes) disclosed in this application as relevant to any embodiment of the invention.

For various example embodiments of the invention, the following is also applicable: a method comprising creating and/or modifying (1) at least one device user interface element and/or (2) at least one device user interface functionality, the (1) at least one device user interface element and/or (2) at least one device user interface functionality based at least in part on data and/or information resulting from one or any combination of methods (or processes) disclosed in this application as relevant to any embodiment of the invention, and/or at least one signal resulting from one or any combination of methods (or processes) disclosed in this application as relevant to any embodiment of the invention.

In various example embodiments, the methods (or processes) can be accomplished on the service provider side or on the mobile device side or in any shared way between service provider and mobile device with actions being performed on both sides.

For various example embodiments, the following is applicable: An apparatus comprising means for performing a method of the claims.

According to some aspects, there is provided the subject matter of the independent claims. Some further aspects are defined in the dependent claims.

Still other aspects, features, and advantages of the invention are readily apparent from the following detailed description, simply by illustrating a number of related embodiments and implementations, comprising the best mode contemplated for carrying out the invention. The invention is also capable of other and different embodiments, and its several details can be modified in various obvious respects, all without departing from the spirit and scope of the invention. Accordingly, the drawings and description are to be regarded as illustrative in nature, and not as restrictive.

### BRIEF DESCRIPTION OF THE DRAWINGS

The example embodiments of the invention are illustrated by way of examples, and not by way of limitation, in the figures of the accompanying drawings:
FIG. 1 is a diagram of a system capable of providing multi-device wearability-aware heat orchestration, according to one example embodiment;
FIG. 2 is a diagram of example devices that can participate in multi-device wearability-aware heat orchestration, according to one example embodiment;
FIGs. 3A and 3B are diagrams illustrating examples of thermal footprints of devices engaged in machine learning workloads, according to one example embodiment;
FIGs. 4A and 4B are diagrams of various components of a device configured to perform multi-device wearability-aware heat orchestration, according to one example embodiment;
FIG. 5 is a flowchart of a process for providing multi-device wearability-aware heat orchestration, according to one example embodiment;
FIG. 6 is a diagram of a user interface for determining user feedback for multi-device wearability-aware heat orchestration, according to one example embodiment;
FIG. 7 is a diagram of hardware that can be used to implement example embodiments; and
FIG. 8 is a diagram of a chip set that can be used to implement example embodiments.

### DESCRIPTION OF SOME EMBODIMENTS

Examples of a method, apparatus, and computer program for providing multi-device wearability-aware heat orchestration, according to one example embodiment, are disclosed in the following. In the following description, for the purposes of explanation, numerous specific details and examples are set forth to provide a thorough understanding of the embodiments of the invention. It is apparent, however, to one skilled in the art that the embodiments of the invention may be practiced without these specific details or with an equivalent arrangement. In other instances, structures and devices are shown in block diagram form to avoid unnecessarily obscuring the embodiments of the invention.

Reference in this specification to "one embodiment", "one example embodiment", "an "embodiment", or "an example embodiment" means that a particular feature, structure, or characteristic described in connection with the embodiment is included in at least one embodiment of the present disclosure. The appearance of the phrase "in one embodiment" or "in one example embodiment" in various places in the specification are not necessarily all referring to the same example embodiment, nor are separate or alternative example embodiments mutually exclusive of other embodiments. In addition, the embodiments described herein are provided by example, and as such, "one embodiment" can also be used synonymously as "one example embodiment." Further, the terms "a" and "an" herein do not denote a limitation of quantity, but rather denote the presence of at least one of the referenced items. Moreover, various features are described which may be exhibited by some embodiments and not by others. Similarly, various requirements are described which may be requirements for some embodiments but not for other embodiments.

As used herein, "at least one of the following: <a list of two or more elements>," "at least one of <a list of two or more elements>," "<a list of two or more elements> or a combination thereof," and similar wording, where the list of two or more elements are joined by "and" or "or", mean at least any one of the elements, or at least any two or more of the elements, or at least all the elements.

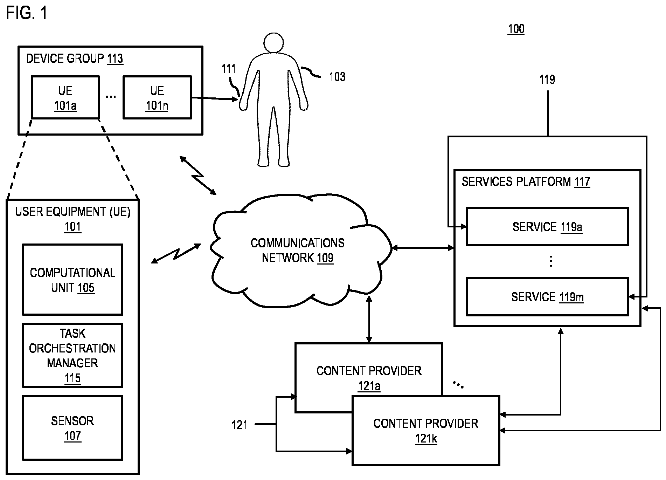

FIG. 1 is a diagram of a system 100 capable of providing multi-device wearability-aware heat orchestration, according to one example embodiment. Currently, user devices (e.g., user equipment (UE) 101a-101n, also collectively referred to as UEs 101 or devices 101, such as smartphones and wearables) increasingly perform a number of processing-heavy and complex on-device tasks in a continuous manner to provide prompt and quality service to users 103, e.g., personalized healthcare solution, fall detection and alert solution, stress monitoring for mental well-being, sleep tracking, and so on. By way of example, a wearable device 101 is an electronic device that is designed to be carried by the user, worn on the body or attached to skin or clothing of a user 103. These devices 101 often have at least computational units 105 and sensors 107 and are connected to a communications network 109 (e.g., the internet) for data exchange between other devices 101 or components of the system 100 (e.g., services 119 and/or content providers 121). Some examples of wearables comprise smartwatches, fitness trackers, virtual reality/augmented reality (VR/AR) headsets, smart rings, smart necklaces, body patches, headphones, and/or the like in any combination thereof.

When these devices 101 perform on-device tasks, they can generate a significant amount of heat, often reaching temperatures between 50°C to 70°C or higher. However, skin-temperatures higher than just, e.g., 44°C can lead to a range of thermal-regulatory disorders and serious health threats (e.g., thermal pain, skin burns, skin aging, etc.). So when wearable devices 101 that reach such temperatures come in contact with or within proximity of the user's skin (e.g., at a skin contact point 111), they can potentially cause heat-related injuries or discomfort. For example, there can be cases where smartwatch users claim that wearables burned their wrists when left on while they were sleeping or when used for prolonged periods of time. Similarly, a temperature monitoring device that is used for continuous monitoring while performing on-device tasks can cause heat-related injuries such as skin burns if the on-device tasks become too intensive.

In one embodiment, a skin contact point 111 between a wearable device 101 and human skin (e.g., of the user 103) refers to the specific area where the device 101 comes into direct or proximate (e.g., within a threshold distance) contact with the wearer's body. This contact point is typically where the device 101 is positioned or attached when it is worn. The design and placement of this contact point can vary depending on the type of wearable device and its intended function. For example, the skin contact point 111 can be located on various parts of the body depending on the design and purpose of the wearable device. Common locations comprise the wrist (for smartwatches and fitness trackers), the chest or torso (for heart rate monitors), the head (for smart glasses or headsets), or other areas where the device can provide optimal functionality.

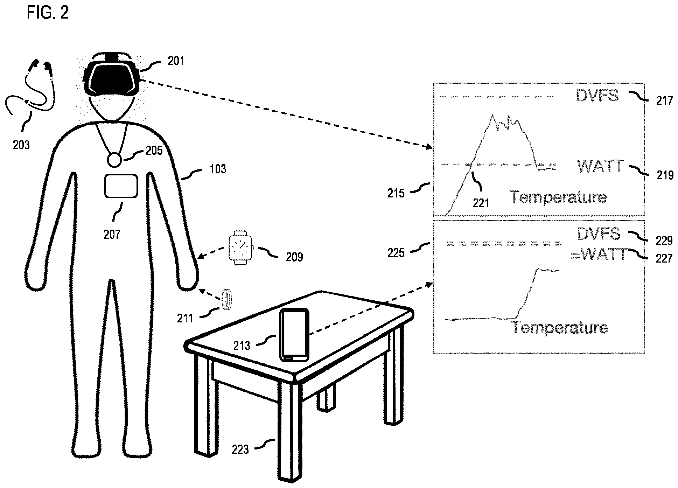

Meanwhile, various smart wearable form factors have been emerging. As illustrated in FIG. 2, a user 103 can have a device group 113 comprising any combination of a AR/VR headset 201, headphones 203, smart necklace 205, body patch 207, smartwatch 209, smart ring 211, smartphone 213, medical sensor device, e.g., electrocardiogram (ECG) monitoring device, blood glucose monitoring device, blood pressure monitoring device, or smart clothing with one or more sensor devices, e.g. smart shoes. It is envisioned that a user 103 may have multiple smart devices 101 worn around, on or inside user's body in the future. This trend amplifies the potential for thermal-related threats. The devices in the device group 113 can communicate with each other via at least one of the following: personal area network (PAN), wireless personal area network (WPAN), body area network (BAN), wireless body area network (WBAN), wireless sensor network (WSN), wireless telecommunication network, short range wireless network (such as wireless local area network (WLAN), Bluetooth, ultra-wide band (UWB), Matter), Peer-to-peer communication (P2P), Device-to-Device communication, or any combination thereof.

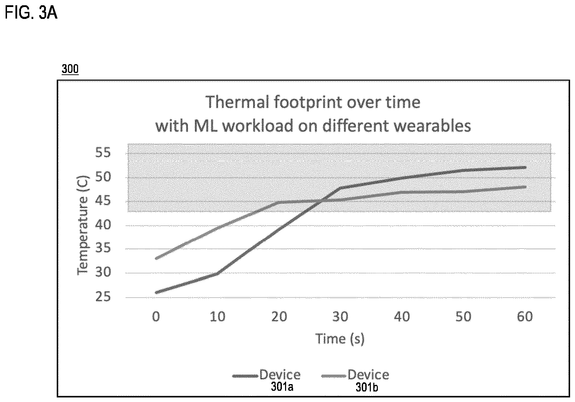

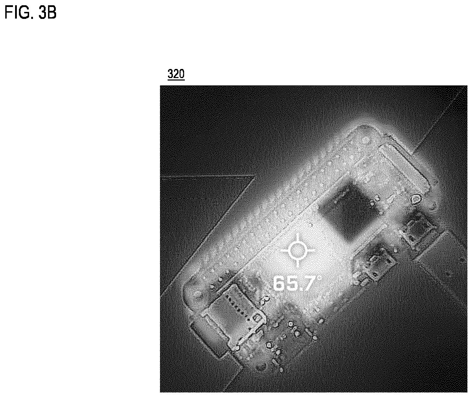

FIGs. 3A and 3B illustrate example heat emissions of small on-body devices 101, for example, when running one or more machine learning (ML) learning and/or inference tasks, according to one example embodiment. The ML (for example, DNN (Deep Neural Network)) learning and/or inference tasks are becoming more commonly performed as on-device tasks and can stress thermal systems of the devices 101. For example, as shown in FIG. 3A, the chart 300 shows the temperatures of two devices 301a and 301b when they are running one or more machine learning inference tasks over an extended period. As seen in the chart 300, the devices 301a and 301b quickly build up heat in a few seconds. More importantly, both devices reach 44 degrees Celsius (which can lead to a range of thermal-regulatory disorders) very quickly (e.g., just in 20 seconds). FIG. 3B illustrates a thermal image 320 of device 301a during the run period of FIG. 3A. The thermal image 320 shows that the device 301a reaches over 65 degrees Celsius during the run period.

Traditional approaches have not considered the problem of (1) thermal management with respect to skin-contact temperature, which is vital because it directly impacts user comfort and safety, (2) ML workload, which is crucial in understanding the heat generation patterns in modern devices that provide many services/tasks based on ML/artificial intelligence (AI), and (3) multi-device environments jointly. In other words, traditional approaches have only considered managing heat of chipsets while neglecting user-perceived heat issues (or how the heat impacts skin) and degraded level of device or system performance and functionality, which pose significant technical challenges.

To address these technical challenges, the system 100 introduces a capability to orchestrate heat across multiple devices to minimize thermal impact of users 103 (e.g., minimize thermal disorders and/or injury to the users 103) while preserving quality and performance of service, by considering device wearability and user-perceived temperature from wearing devices 101. In one embodiment, the system 100 (e.g., via a task orchestration manager 115) introduces a capability to provide heat management that distinguishes itself from traditional solutions. The various embodiments for heat management described herein consider both device wearability and user-perceived heat for multi-device heat orchestration. In one example, embodiment, the system 100 uses the following techniques to provide multi-device wearability-aware heat orchestration:
1. Skin-Contact Temperature Modeling: This aspect involves profiling and modeling the relationship between device task workload and actual temperature measured at the users' skin (e.g., at skin contact point 111).
2. Wearability Prediction: Using various sensors 107 (e.g., IMU and proximity sensors, temperature sensor), the system 100 predicts the wear status of one or more or each device (e.g., on-body, off-body, part of body, etc.). As used herein, the term "wear status" refers to the indication of whether (e.g., on-body, off-body) and how (e.g., which body part, direct contact, over clothing, etc.) the device is being worn by a user. It provides information about the physical state or position of the wearable in relation to the user's body. This status can encompass various aspects such as whether the device is currently worn, how it is positioned (e.g., on the wrist, clipped to clothing), and potentially additional details such as orientation or motion.
3. Wearability-Aware Task Orchestration: one or more tasks are orchestrated across multiple devices, considering skin temperature and wearability, and additionally in some implementations also performance of the system 100. A dynamic wearability-aware temperature threshold (WATT) (also referred to as a wearable temperature threshold or thermal budget of a device 101 and/or the system 100) is used to regulate device temperatures, with tasks distributed to avoid a situation where the predicted skin temperature at the skin contact point (also referred to herein as the "predicted skin-contact temperature") is higher than WATT. As used herein, the term "task orchestration" refers to delegating, assigning, scheduling, and/or monitoring computational tasks (e.g., one or more ML inference or training tasks and/or another type of compute tasks, for example, encoding/decoding audio and/or video content or streaming, and/or transmitting audio and/or video content or streaming) across multiple wearable devices 101 and/or other related devices (e.g., other devices in a device group 113 associated with a user 103 comprising but not limited to non-wearable devices such as smartphones, personal computers, tablets, televisions, displays, cloud computing devices, etc.).

Returning to the example of FIG. 2, the devices 201-213 are part of a device group (e.g., a device group 113) that can implement the multi-device wearability-aware heat orchestration, according to one example embodiment. In this example, given high heat generation from the VR headset 201 running heavy vision applications (e.g., using one or more ML inference tasks for computer vision applications, such as decoding audio and/or video stream), the skin-contact temperature is over WATT (e.g., the wearable temperature threshold that will trigger task orchestration to reduce the skin-contact temperature of the VR headset 201). This is illustrated in VR headset temperature chart 215 where the DVFS line 217 indicates the temperature at which the VR Headset 201 will thermally throttle its CPU/GPU to reduce heat and the WATT line 219 is set to the maximum temperature that does not cause thermal injury or discomfort to the user's skin that is in contact with the VR headset 201. Additionally or alternatively to the skin-contact temperature, the multi-device wearability-aware heat orchestration system can also measure and consider temperature inside of the VR headset 201, e.g. in the space between user's eyes and a display of the headset 201.

At the point in time 221 that the VR headset temperature is greater than the WATT line 219, the multi-device wearability-aware heat orchestration system is triggered. Then, the computational tasks or workload (e.g., one or more ML training or inference tasks, encoding/decoding audio and/or video content, etc.) of the VR headset 201 are distributed to other devices considering their wearability and accordingly their respective thermal budgets. In this case, the smartphone 213 has the greatest thermal budget (e.g., highest WATT) because it is placed on, for example, a table 223 and/or not in direct contact or close proximity to the user's skin. Accordingly, as illustrated in the smartphone 213 temperature chart 225, the WATT line 227 for the smartphone 213 can be set at the maximum temperature at which it will not thermal throttle to reduce performance (e.g., just below the DVFS line 229). The system 100 redistributes the computational tasks or workload (e.g., the one or more ML inference tasks) of the VR headset 201 to the smartphone 213 which reduces the VR headset temperature below its WATT line 219, while the temperature of the smartphone 213 increases but remains below both its WATT line 227 and DVFS line 229. Accordingly, after the heat orchestration to redistribute tasks, the skin-contact temperature and/or the inside temperature of in the VR headset 201 drops down under WATT to advantageously reduce the potential for thermal discomfort and/or injury to the user.

By way of example, the various embodiments of multiple-device wearability-aware heat orchestration described herein have a variety of applications comprising but not limited to:
1. Enhanced Heat Management for Collaborative training of ML Frameworks (e.g., Federated Learning): The various embodiments described herein are beneficial for collaborative machine learning frameworks like Federated Learning. By intelligently distributing workloads and managing thermal impacts across a network of devices, it ensures the seamless operation of such frameworks, facilitating efficient model training without overheating concerns and compromising performance of the learning framework.
2. Advanced Heat Management for Sensory Tasks with Data Collection and Inference: The various embodiments described herein are applicable to sensory tasks that involve both data collection and inference processes. It optimizes the thermal profile of devices engaged in sensory tasks, guaranteeing stable performance while preserving user comfort and device longevity.
3. Elevating User Experience in Wearable Devices: The various embodiments described herein improve user comfort and safety in wearable devices by dynamically adjusting thermal management based on wearability factors. Users can enjoy extended usage and uncompromised performance without discomfort or overheating issues.
4. VR/AR Applications: As virtual reality/augmented reality (VR/AR) headsets become more popular and demanding with higher workloads, such as machine learning for 4K screens, the various embodiments described herein play an important role in reducing heat generated by the AR/VR devices. This ensures a comfortable and immersive AR/VR experience without uncompromised performance.
5. Enhanced Baby or Patient Monitoring Systems: The various embodiments described herein hold immense potential for applications in baby/patient monitoring systems, especially beneficial for caregivers who need to monitor the health of babies/patients or individuals with limited communication abilities. By effectively managing device heat, it contributes to the safety and well-being of vulnerable individuals without uncompromised performance.

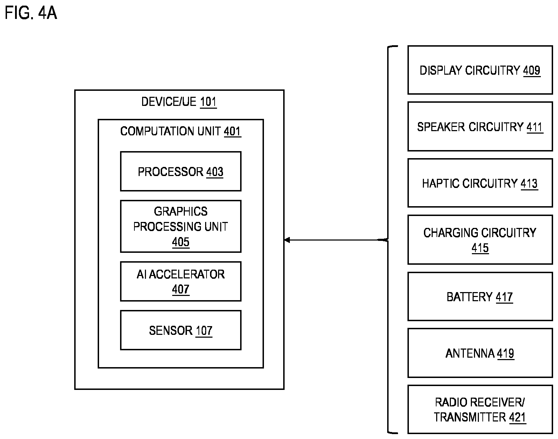

In one embodiment, the devices/UEs 101 that participate in heat orchestration comprise various components comprising one or more device computation units, e.g., one or more processors, and other components, e.g., one or more encoders or decoders, that can generate heat from performing on-device tasks. FIG. 4A is a diagram of a device/UE 101 configured to perform multi-device wearability-aware heat orchestration, according to one example embodiment. As shown, the device/UE 101 comprises a computation unit 401. The computation unit 401 refers to a component that is responsible for performing computational tasks (e.g., one or more ML inference or training tasks, etc.). As used herein, a "computational task" refers to any task or problem that can be solved or processed using computational methods or algorithms. In other words, it is a task or problem that can be broken down into a series of steps or operations that a computer can execute to produce a desired outcome. As shown, in one embodiment, the computation unit 401comprises: (1) one or more processor 403 or CPU that is the component responsible for executing instructions and performing calculations in a computer such as but not limited to arithmetic operations, logic operations, and data movement; (2) one or more graphics processing units (GPU) 405 comprising highly parallel processors capable of performing many floating-point calculations simultaneously and e.g. used for rendering graphics, image processing, scientific simulations, machine learning modeling, and/or the like; (4) one or more artificial intelligence (AI) accelerator 407 such as a neural processing unit (NPU)/tensor processing unit (TPU) comprising application-specific integrated circuits (ASICs) or equivalent circuitry for accelerating machine learning workloads such as but not limited to matrix multiplication; and (5) one or more sensors 107 performing a variety of functions comprising but not limited to temperature measurement (e.g., skin temperature measurement), biometric monitoring, activity tracking, sleep monitoring, environmental monitoring, location tracking, gesture recognition, biofeedback measurement, fall detection, and/or the like.

In addition to the computation unit 401, the device/UE 101 may also comprise one or more of: (1) display circuitry 409, such as video encoder, to translate signals into visual output on a screen of the device/UE 101; (2) speaker circuitry 411, such as audio encoder, to convert signals into sound waves for reproduction of audio output; (3) haptic circuitry 413 to generate tactile feedback, such as vibrations or sensations, enhancing user interaction and providing sensory cues; (4) charging circuitry 415 to regulate the flow of electrical current to safely charge the battery of a device; (5) battery 417 to store and provide electrical energy to a device/UE 101; (6) antenna 419 to transmit and receive radio frequency (RF) signals; and (7) radio receiver/transmitter 421 to capture and convert RF signals receive/sent by the antenna 419 into electrical signals enabling the device/UE 101 to extract information/data from wireless transmissions. It is noted that the above components are provided by way of illustration and not as limitations. It is contemplated that in addition or alternatively, any other combination of heat generating components can be included in the device/UE 101 to perform on-device tasks that can be orchestrated among multiple devices 101 according to the various embodiments described herein.

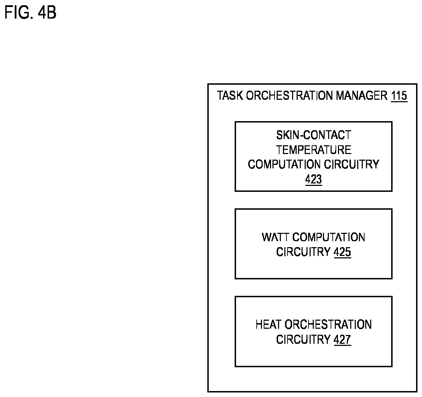

In one embodiment, for example, in FIG. 4B, the various embodiment of multiple-device wearability-aware heat orchestration is performed using a task orchestration manager 115 comprising: (1) skin-contact temperature computation circuitry 423 to process input data, applying mathematical transformations, and generating output predictions or classification based on learned patterns and parameter (e.g., via machine learning or equivalent computer algorithms); (2) wearable temperature threshold circuitry 425 to use sensors or components integrated into devices/UEs 101 to assess various factors to determine if the device/UE 101 is being worn or is in contact with the wearer; and (3) heat orchestration circuitry 427 to manage and coordinate the allocation and execution of tasks across a device group 113 (e.g., interconnected devices associated with a particular user of a wearable device 101).

It is contemplated that the functions of the components of the device/UE 101 and/or system 100 or any of their components described above may be combined or performed by other components or means of equivalent functionality. The above presented components comprise means for performing the various embodiments and can be implemented in a circuitry, a hardware, a firmware, a software, a chip set, or in any combination thereof.

As used in this application, the term "circuitry" may refer to one or more or all of the following:
(a) hardware-only circuit implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (comprising digital signal processor(s)), software, and memory(ies) that work together to cause an apparatus, such as a mobile phone or server, to perform various functions) and
(c) hardware circuit(s) and or processor(s), such as a microprocessor(s) or a portion of a microprocessor(s), that requires software (e.g., firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device (e.g., the device 101) or a similar integrated circuit in server, a cellular telecom network device, or other computing or network device.

In another embodiment, one or more of the components of the device/UE 101 and/or system 100 may be implemented as a cloud-based service, local service, native application, or in any combination thereof. In one embodiment, as shown in FIG. 1, the device/UE 101 also has connectivity via the communications network 109 to a service platform 117 which comprises one or more services 119a-119m (also collectively referred to as services 119) (e.g., health and wellness service, ML training and/or inferencing services, multimedia service, AR/VR service, audio or video streaming service or any other service), one or more content providers 121a-121k (also collectively referred to as content providers 121) (e.g., online content retailers, video streaming services, audio streaming services, public databases, etc.). In one embodiment, the service platform 117, services 119, and/or content providers 121 alone or in combination can create computational workloads on the devices/UEs 101 that result in heat generation. This heat generation can then be addressed using various embodiments of multiple-device wearability-aware heat orchestration described herein. The functions of the system 100 and its components are discussed with respect to the figures below.

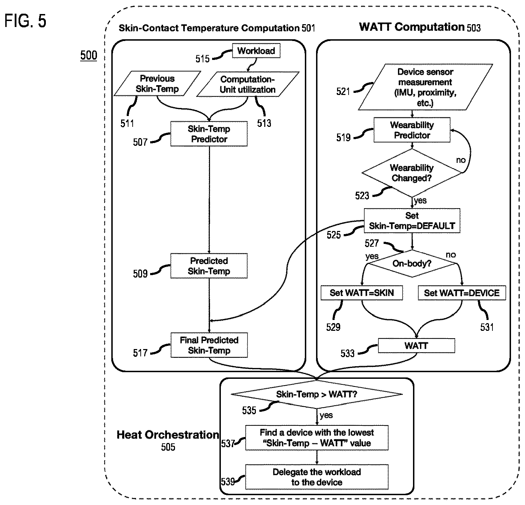

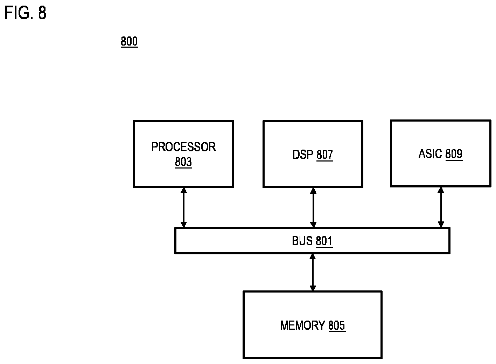

FIG. 5 is a flowchart of a process 500 for providing multi-device wearability-aware heat orchestration, according to one example embodiment. In one example, the task orchestration manager 115, system 100 and/or any of their components (such as the user equipment 101, the service 119 or the content provider 121) may perform one or more portions of a process 500 and may be implemented in/by various means, for instance, one or more chip sets comprising a processor and a memory as shown in FIGs. 8 or 9 or in a circuitry, hardware, firmware, software, or in any combination thereof. In one example embodiment, the circuitry comprises but is not limited to any component discussed with respect to FIGs. 4A and 4B (e.g., modeling circuitry, detection circuitry, heat orchestration circuitry, etc.). As such, task orchestration manager 115, system 100 and/or any associated component, apparatus, device, circuitry, system, computer program product, and/or non-transitory computer readable medium, or any combination thereof, can provide means for accomplishing various parts of the process 500, as well as means for accomplishing embodiments of other processes described herein. Although the process 500 is illustrated and described as a sequence of steps, it is contemplated that various embodiments of the process 500 may be performed in any order or combination and need not include all of the illustrated steps.

In one embodiment, the process 500 consists of three components or sub-processes: (1) Skin-Contact Temperature computation 501, (2) WATT computation 503, and (3) Heat orchestration 505. For example, the Skin-Contact Temperature computation 501 estimates the temperature at the skin-contact point 111 of a wearable device 101 based on one or more computation task workloads 515 (e.g., ML tasks). The Wearability Aware Temperature Threshold (WATT) computation 503 estimates the wear status of a device 101 and dynamically configures the wearable temperature threshold, WATT 533. Lastly, the heat orchestration 505 manages the computational workload (e.g., ML workload) between the devices 101 within a device group 113 once a wearable device 101's temperature exceeds the WATT. The following section describes the process 500 in detail.

In one embodiment, the skin-Contact Temperature computation 501 comprises the following processes. In one embodiment, the "Skin-Contact Temperature Predictor 507" (e.g., comprising or otherwise skin-contact temperature computation circuitry 423) estimates the temperature at the skin-contact point 111 of a wearable device 101 (e.g., predicted skin temperature 509). In other words, the skin-contact temperature computation circuitry 423 determines a predicted skin temperature 509 at a skin contact point 111 of a wearable device 101. This estimation, for instance, relies on previously measured temperatures (e.g., previous skin temperature measurements 511) and computation-unit utilizations 513 calculated from the computational workload 515. The computation-unit utilization 513 is calculated by comparing the actual worked performed by the computation-unit 401 to its maximum capacity within a specific timeframe expressed, for instance, as a percentage. Additionally or alternatively, the computation-unit utilization 513 can be calculated by considering estimated future computational workload/tasks 515 to be performed by the computation-unit 401. In other words, the skin-contact temperature computation circuitry 423 models a relationship between the computational workload 515 of the wearable device 101 and a skin temperature measured at the skin contact point 111. Modeling, for instance, is the process of creating a representation of a system, phenomenon, or behavior. It involves using mathematical equations, algorithms, and data to create a simplified version of reality that can be used to understand, analyze, and make predictions about complex systems. Modeling can be done using a variety of techniques, comprising statistical modeling, computer simulations, and machine learning. In one embodiment, the final predicted skin-contact temperature 517 reflects the predicted skin-contact temperature 509 as potentially affected by the WATT computation process 503 as explained below.

In one embodiment, the skin-contact temperature predictor 507 is based on modeling device-specific skin-contact temperatures. Given that different devices 101 have unique hardware compositions and form factors, and/or unique computer software/program installations, for example, one or more ML learning and/or inference programs, (e.g., see FIG. 2), the skin-contact temperature predictor 507 models a different skin-contact temperature predictor for one or more or each specific device 101. Modeling a different skin-contact temperature predictor for one or more or each specific device involves tailoring predictive algorithms to account for unique characteristics, such as sensor precision, placement, material properties, computer software/program installations, type and/or construction of one or more DNN models, or any combination thereof, inherent to individual wearable devices. By customizing predictors for one or more or each device, the model can optimize accuracy and account for variations in temperature readings, ensuring reliable estimation of skin-contact temperatures specific to one or more or each device's design and usage context.

In one embodiment, the modeling process is as follows:
(1) Adaptive Task-based Thermal Profiling: The skin-contact temperature predictor 507 executes a diverse set of tasks, such as ML inference and/or training, audio sensing, IMU sensing, and data processing, across various computation units 401 (e.g., GPU, CPU, AI accelerator, IMU, BLE, etc.). Each task and computation unit 401 has unique thermal characteristics. For instance, an ML inference and/or training task will increase the temperature of the processor 403, GPU 405 and/or AI accelerator 407, while sensor-based tasks will raise the temperature of the respective sensor 107. To capture these thermal profiles, the process 500 can use a temperature sensor. Thus, in one embodiment, the device 101 may comprise or have access to means for determining a measured skin temperature at the skin contact point 111. If the wearable device 101 already has an integrated temperature sensor 107 for skin monitoring, e.g., a sensor-equipped smartwatch, the thermal profiling can run at the software level and run even at runtime. Otherwise, the profiling can be done at offline using an external, high-precision temperature sensor 107. In this way, the predicted skin temperature is further based on the measured skin temperature.
(2) Context-Aware Skin-Contact Temperature Prediction Model: In one embodiment, the modeling of the skin temperature is based on a predictive model that estimates skin-contact temperature based on current and/or predicted utilization of the computational unit 401, while also incorporating real-time contextual data such as user activity level, ambient temperature, and humidity. In addition, contextual data affecting skin temperature at a wearable device's skin contact point 111 further comprises clothing insulation, body position, skin properties, external factors like sunlight and humidity, health conditions, and emotional states. Monitoring these factors enables personalized adjustments to optimize user comfort and device performance. Incorporating contextual data into the modeling can be achieved, for instance, through a hybrid machine learning model that combines traditional techniques like linear regression with advanced neural network layers specifically designed to interpret contextual data. To support this embodiment, the skin-temperature predictor 507 can further comprises means (e.g., sensors 107) for determining contextual data associated with the wearable device, a user of the wearable device, an environment in which the wearable device is operating, or a combination thereof, such that the predicted skin temperature is further based on the contextual data.
(3) User Feedback Loop for Calibration: In one embodiment, the skin-contact temperature computation 501 comprises a user feedback mechanism that allows users to input their perceived temperature comfort level. This data is used to fine-tune the predictive model in real-time, making it more accurate and personalized. By collecting users' subjective comfort ratings, the model can correlate perceived comfort with actual skin temperature data collected from wearable sensors or predictions. This feedback loop allows the model to adjust its algorithms, parameters, and thresholds to better align predicted skin temperatures with users' comfort preferences, resulting in a more accurate and personalized temperature estimation tailored to individual user comfort levels.

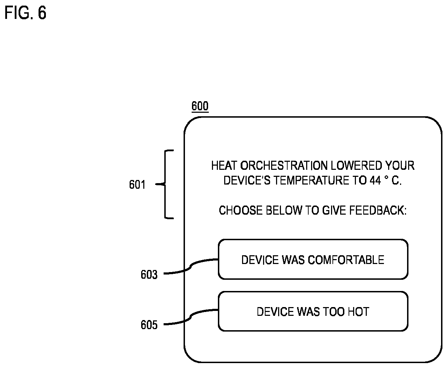

Thus, the skin-contact temperature computation 501 can comprise means for determining a user's perceived temperature comfort level (e.g., a user interface for collecting such input). For example, FIG. 6 is a diagram of a user interface 600 for determining user feedback for multi-device wearability-aware heat orchestration, according to one example embodiment. In this example, an embodiment of the heat orchestration approach described herein has been used to lower a wearable device 101's temperature to a default skin-safe temperature of 44 ° C. The user interface 600 can then be presented to the user to obtain feedback on the user's perceived comfort level. As shown, the user interface 600 presents a message 601, "HEAT ORCHESTRATION LOWERED YOUR DEVICE'S TEMPERATURE TO 44 ° C," and provides a user interface element 603 for the user to indicate that the "DEVICE WAS COMFORTABLE" and a user interface element 605 for the user to indicate that the "DEVICE WAS TOO HOT." The user can interact with either user interface element 603 or 605 to provide feedback.

Example pseudo code for implementing the skin-contact temperature computation 501 is provided in Tables 1-8 below. It is noted that the pseudo code is provided by way of illustration and not as limitations. It is contemplated that equivalent code or procedures can be used according to the various embodiments described herein. In one embodiment, the skin-contact temperature computation circuitry 423 or equivalent device/apparatus can execute computer code or instructions derived from the pseudo code to perform skin-contact temperature computation 501 according to various embodiments described herein.

Table 1 below illustrates pseudo code for initializing variables and data structures for skin-contact temperature computation 501, according to one example embodiment.

**Table 1**

| |
|---|
| ```
 # Initialize variables and data structures
 initialize skin_contact_temperature_models = {} # Dictionary to store device-specific
 models
 initialize user_feedback_data = {} # Dictionary to store user feedback data
``` |

The example pseudo code of Table 1 sets up the foundation for storing information that will likely be used in the system 100 to manage heat or thermal comfort as follows:
(skin_contact_temperature_models): This creates a dictionary where the code can store different models related to skin-contact temperature. These models, for instance, can contain formulas or patterns used to predict how hot a user's skin will get in various conditions, with different models potentially assigned to different devices.
(user_feedback_data): This creates another dictionary to hold direct feedback from the user about their comfort level. This might comprise the user providing input to say things like "My skin is too hot" or "This feels fine." This feedback can help the system tailor heat management to the individual's preferences.

Table 2 below illustrates pseudo code for the main logic of skin-contact temperature computation 501, according to one example embodiment.

**Table 2**

| |
|---|
| ```
 # Main logic
 while True:
   # Continuously monitor and profile devices
   for device in devices:
     adaptive_task_based_thermal_profiling(device)
   # Continuously collect user feedback
   for device in devices:
     user_feedback = get_user_feedback(device)
     if user_feedback:
       collect_user_feedback(device, user_feedback)
   # Predict skin-contact temperatures for devices in use
   for device in devices in use:
``` |
| ```
     utilization = get_computational_unit_utilization(device)
     context_data = get_contextual_data(device)
     predicted_temperature = predict_skin_contact_temperature(device, utilization,
 context_data)
     if predicted_temperature:
       display_temperature(device, predicted_temperature)
   # Calibrate models with user feedback
   for device in devices:
     calibrate_model_with_user_feedback(device)
``` |

The example pseudo code of Table 2 represents the main logic for the system 100 to monitor devices (e.g., UEs 101 with device group 113) to prevent them from creating too much heat that could injure the user's skin or cause heat-related discomfort. The pseudocode works in a continuous loop with the following main actions:
Device Monitoring: It looks at each device in the system (devices) and runs a function called adaptive_task_based_thermal_profiling. This function analyzes how hot the device is getting, which tasks it is running, and determines how those tasks contribute to the device's temperature.
User Feedback: It then gathers feedback (get_user_feedback) from the user for each device. If the user provides input, this feedback is stored (collect_user_feedback) for later use.
Prediction Time: The pseudocode focuses on devices_in_use (currently active devices). It figures out how much processing power each device is using (get_computational_unit_utilization) and gathers other relevant information (get_contextual_data) like its location or surroundings. Using all this, it tries to predict how hot the user's skin temperature at the skin contact point of the device might get (predict_skin_contact_temperature). If it makes a prediction, it can display this temperature to the user (display_temperature).
Applying User Feedback: Finally, the code takes all the user feedback it has collected and uses the feedback to improve its temperature prediction models (calibrate_model_with_user_feedback). This means the system is learning from the user's preferences to perform task orchestration.

Table 3 below illustrates pseudo code for performing adaptive task-based thermal profiling for skin-contact temperature computation 501, according to one example embodiment.

**Table 3**

| |
|---|
| ```
 # Function to perform Adaptive Task-based Thermal Profiling
 function adaptive_task_based_thermal_profiling(device):
   # Collect temperature data from integrated or external sensors
   if device has_integrated_temperature_sensor:
     temperature_data = read_internal_temperature_sensor()
   else:
     temperature_data = read_external_temperature_sensor()
``` |

The example pseudo code of Table 3 provides an adaptive_task_based_thermal_profiling function that is responsible for determining the temperature at the skin contact point of a device. The approach illustrated in the pseudo code is as follows. First, the pseudo code determines whether the device has an integrated sensor. If the answer is yes (device.has_integrated_temperature_sensor), the code can directly read the temperature data from the device's internal sensor (read_internal_temperature_sensor). If the device does not have a built-in sensor, the code assumes there is an external temperature sensor attached or otherwise accessible. It then reads the temperature data from this external sensor (read_external_temperature_sensor).

Table 4 below illustrates pseudo code for profiling thermal characteristics for each task and computation unit 401 for skin-contact temperature computation 501, according to one example embodiment.

**Table 4**

| |
|---|
| ```
 # Profile thermal characteristics for each task and computation unit
 for task in device.tasks:
     for computation _unit in device.computation _units:
       thermal_profile = profile_thermal_characteristics(task, computation_unit,
 temperature_data)
       store_thermal_profile(device, task, computation_unit, thermal_profile)
``` |

The example pseudo code of Table 4 determines the thermal characteristics of each task and computation unit of a device. The code starts by looking at each task (device.tasks) the device is handling. For each task, it then examines all the individual computation units (device.computation_units) involved in running that task. The code calls a function called profile_thermal_characteristics that analyzes how a specific task running on a specific computation unit affects the skin temperature at the skin contact point of the device.

Table 5 below illustrates pseudo code for storing thermal profiles for each device 101, task, and computation unit 401 for skin-contact temperature computation 501, according to one example embodiment.

**Table 5**

| |
|---|
| ```
 # Function to store thermal profiles for each device, task, and computation unit
 function store _thermal_profile(device, task, computation _unit, thermal_profile):
   skin_contact_temperature_models[device][task][computation_unit] = thermal_profile
``` |

Table 6 below illustrates pseudo code for predicting skin-contact temperature for skin-contact temperature computation 501, according to one example embodiment.

**Table 6**

| |
|---|
| ```
 # Function to predict skin-contact temperature
 function predict_skin_contact_temperature(device, computational_unit_utilization,
 context _data):
   if device in skin_contact_temperature_models:
     model = skin_contact_temperature_models[device]
     prediction = calculate_skin_contact_temperature(model,
 computational_unit_utilization, context_data)
     return prediction
   else:
     return None # Model not available for the device
``` |

The example pseudo code of table 6 calls a predict_skin_contact_temperature function to calculate how hot a device might get against a user's skin. It first checks if a predictive model exists for the device in its database (skin_contact_temperature_models). If a model is found, it feeds that model, the device's current workload (computational_unit_utilization), and environmental factors (context_data) into a calculation to estimate the surface temperature. If the device does not have a model on file, the function returns None, indicating it lacks the necessary information to make a prediction.

Table 7 below illustrates pseudo code for collecting user feedback on perceived temperature comfort level for skin-contact temperature computation 501, according to one example embodiment.

**Table 7**

| |
|---|
| ```
 # Function to predict skin-contact temperature
 function collect_user_feedback(device, user_feedback):
   user_feedback_data[device] = user_feedback
``` |

Table 8 below illustrates pseudo code for fine-tuning the predictive model based on user feedback for skin-contact temperature computation 501, according to one example embodiment.

**Table 8**

| |
|---|
| ```
 # Function to fine-tune the predictive model based on user feedback
 function calibrate_model_with_user_feedback(device):
   if device in user_feedback_data:
     user_feedback = user_feedback_data[device]
     fine_tune_model(device, user_feedback)
``` |

The example pseudo code of Table 8 calls calibrate_model_with_user_feedback function to personalize the system's temperature prediction models by incorporating direct feedback from the user. First, it checks if there is any recorded user feedback (user_feedback_data) related to the specific device. If feedback exists, it retrieves that feedback and passes it, along with the device information, to a function called fine_tune_model. This fine_tune_model function can use the user's input (e.g., "too hot," "feels okay," etc.) to adjust the prediction model for that device, aiming to make its future temperature estimations more aligned with the individual user's preferences.

In the WATT computation 503, a wearability predictor 519 (or equivalent wearable temperature threshold computation circuitry 425) estimates the wear status (e.g., on wrist, hand, head, ear, etc.) of a device 101 by, for instance, utilizing the embedded sensors (IMU, proximity sensors, etc.) to determine device sensor measurement 521. Motion, proximity, temperature, pressure, and orientation sensors provide data on movement patterns, skin contact, temperature changes, and device orientation relative to the body, allowing inference of whether the device is being worn and its position. Thus, the wearability predictor 519 and/or equivalent wearable temperature threshold computation circuitry 425 comprise means for determining a wear status of the wearable device. As previously noted, the wear status of a wearable device 101 refers to its current state of being worn (e.g., on-body) or not worn (e.g., off-body) by the user. It can comprise information about how the device is positioned on the body, whether it is in contact with the skin, and/or any relevant contextual details such as orientation or movement.

In one embodiment, the WATT computation 503 is triggered based on a wearability change 523 (e.g., a change in the wear status of the device 101). For example, if the wear status did not change, the wearability predictor 519 waits until the status changes.

At process 525, if the wear status did change, that means the thermal impact of the device 101 to the previous skin contact may no longer have effect. Then, the wearability predictor 519 sets the Skin-Contact Temperature (Skin-Temp) to a default value (DEFAULT). In one embodiment, the default temperature can be standard human body temperature or a measured body temperature of the wearer. In one embodiment, this default temperature can also be provided to the skin-contact temperature computation 501 to determine a final predicted skin-contact temperature 517 from the predicted skin-contact temperature 509.

At process 527, the wearability predictor 519 predicts that the wear status of the device 101 has changed, and then determines the new wear status of the device. As discussed above, in one embodiment, the wear status comprises an on-body status that indicates that the wearable device is being worn, and an off-body status that indicates that the wearable device is not being worn. If the predicted wear status is on-body (e.g., based on determining that the wear status is the on-body status), the wearability-aware temperature threshold (WATT) (or simply wearable temperature threshold) is set to (1) the maximum temperature that does not cause thermal disorders or injury, e.g., at the skin contact point 111, and/or (2) the maximum comfortable usage temperature specified by the user. This value is referred to as SKIN. In one embodiment, the SKIN value can change according to different body positions of the wearable device 101 at process 529. Thus, the maximum temperature, the maximum comfortable temperature, or a combination thereof is specified separately for different body parts on which the wearable device can be worn.

If the predicted wear status of the device 101 is off-body (e.g., based on determining that the wear status is the off-body status), the WATT or wearable temperature threshold is set to the maximum temperature that the device 101 can operate without degraded performance or at which the wearable device 101 can operate without reducing a quality of service (e.g., caused by thermal throttling) at process 531. This value is referred to as DEVICE and can be defined by the system 100 based on, for instance, the traditional dynamic voltage frequency scaling (DVFS) algorithm. The output of the WATT computation 503 is then provided as the WATT or wearable temperature threshold 533.

In one embodiment, wearable temperature threshold or WATT is a dynamic threshold that triggers heat orchestration 505. If the current predicted temperature measured at a device 101 (e.g., final predicted skin-contact temperature 517) is higher than this threshold (e.g., WATT), the orchestration runs and one or more computational tasks are distributed to other devices 101 to reduce the thermal burden of the device 101 as explained in more detail further below.

Example pseudo code for implementing the WATT computation 503 is provided in Tables 9-11 below. It is noted that the pseudo code is provided by way of illustration and not as limitations. It is contemplated that equivalent code or procedures can be used according to the various embodiments described herein. In one embodiment, the WATT computation circuitry 425 or equivalent device/apparatus can execute computer code or instructions derived from the pseudo code to perform WATT computation 503 according to various embodiments described herein.

Table 9 below illustrates pseudo code for initializing variables for WATT computation 503, according to one example embodiment.

**Table 9**

| |
|---|
| ```
 # Initialize variables
 initialize current _wear_status = None # Tracks the current wear status
 initialize skin_contact_temperature = DEFAULT # Initialize with default temperature
 initialize WATT = DEFAULT # Initialize WATT with the default temperature
``` |

Table 10 below illustrates pseudo code for initializing constants for WATT computation 503, according to one example embodiment.

**Table 10**

| |
|---|
| ```
 # Constants
 SKIN_THRESHOLD = 44 # The maximum temperature that does not cause injury to
 human skin
 DEVICE THRESHOLD = SYSTEM DEFINED THRESHOLD # The maximum
 temperature that the device can endure before degrading performance
``` |

Table 11 below illustrates pseudo code for the main loop for WATT computation 503, according to one example embodiment.

**Table 11**

| |
|---|
| ```
 # Main loop for WATT computation
   # Check for changes in wear status
   new_wear_status = estimate_wear_status()
   if new_wear_status != current_wear_status:
     # Wear status has changed
     current_wear_status = new_wear_status
     if current_wear_status == "on-body":
       # Set WATT to SKIN threshold if the device is on the body
       WATT = SKIN_THRESHOLD
       skin_contact_temperature = DEFAULT # Reset skin-contact temperature
     elif current_wear_status == "off-body":
``` |
| ```
       # Set WATT to DEVICE threshold if the device is off the body
       WATT = DEVICE_THRESHOLD
       skin_contact_temperature = DEFAULT # Reset skin-contact temperature
``` |

The example pseudo code of Table 11 is the main logic for dynamically calculating WATT. The code checks if the "wear status" of a device has changed (e.g., whether it is on a user's body or not). If a change is detected, the code updates the current_wear_status and recalculates WATT accordingly. When the device is on the body (on-body), WATT is set to SKIN_THRESHOLD to protect the user. If the device is not on the body (off-body), WATT is set to DEVICE_THRESHOLD, allowing for potentially higher temperatures since the main concern is the maximum temperature that the device can operate without degrading performance (e.g., performance drops by more than a threshold level).

In one embodiment, after the skin-contact temperature computation 501 and WATT computation 503, the heat orchestration 505 can be performed. For example, with the final predicted skin-contact temperature 517 from Skin-Contact Temperature Computation 501 and WATT 533 from WATT Computation 503, Heat Orchestration 505 determines whether the final predicted skin-contact temperature 517 is higher than the wearable temperature threshold, WATT 533 at process 535.

At step 537, if the final predicted skin-contact temperature 517 is higher than WATT 533, the heat orchestration circuitry 427 determines one of the devices 101 of a device group 113 associated with a user with the highest thermal budget, e.g., the lowest value for the device's respective WATT value is selected as a target device. Accordingly, the heat orchestration circuitry determines another wearable temperature threshold and/or one or more respective wearable temperature thresholds for one or more other devices 101 or other candidate devices 101 of the user's device group 113, and then selects at target device 101 (e.g., at least one device to which at least one portion of the computational workload of the wearable device 101 is delegated) based on the one or more respective wearable temperature thresholds. In one embodiment, the selected at least one or more other target devices 101 has a maximum threshold, e.g. the highest thermal budget, of the one or more respective wearable temperature thresholds of the other devices 101 of the device group 113. When determining one or more of the wearable temperature thresholds and/or the one or more respective wearable temperature thresholds for the one or more other devices 101 or the other candidate devices 101 the heat orchestration circuitry 427 may also consider for example, heat generation effects of one or more computation task workloads 515, such as the one or more ML training or inference tasks, the encoding/decoding audio and/or video content, etc. or any combination thereof, which are currently executed or planned to be executed in any of the UEs 101a-101n.

The wearable device 101 then delegates one or more parts of a specific workload or whole specific workload to the selected one or more target devices 101 via at step 539, for instance, over wireless device-to-device (D2D) communication, and the one or more target devices 101 runs the workload instead. The one or more delegated parts of a specific workload can be, for example, one or more parts of a specific DNN model. Device-to-device communication can be utilized to delegate a computational workload among multiple devices by establishing communication channels between them. Through protocols such as but not limited to Bluetooth, Wi-Fi Direct, or ad-hoc networks, devices can coordinate tasks, distribute data, and synchronize computations. By way of example, the delegating 539 computational workload between devices involves breaking down the workload into smaller tasks and distributing them among multiple devices 101. This can be achieved through communication protocols like message passing or remote procedure calls, where tasks are sent to remote devices for execution. Upon completion, results are aggregated and processed further if needed. This allows for collaborative processing, where tasks are divided and allocated based on device capabilities, optimizing resource utilization, and performing heat orchestration. This way, devices can collaboratively reduce the thermal impact to the user.

In one embodiment, the inputs of the heat orchestration 505 are a set of tasks (T) (e.g., comprising the computational workload of a wearable device 101 to be distributed such as one or more ML inference and/or training tasks), a set of devices 101 (D) (e.g., device group 113), skin-contact temperatures, and WATTs. The output is a set of mapping (Task, Device) indicating which computational task is assigned to which device 101, or which one or more computational tasks are assigned to which one or more devices 101.

In one embodiment, the computational workload is distributed across at least one of the components of FIGs. 4A and 4B comprising but not limited to a central processing unit 403 (CPU), a graphics processing unit 405 (GPU), an artificial intelligence accelerator 407, an inertial measurement unit, a wireless transmitter, or a combination thereof of the wearable device. Accordingly, the predicted skin temperature used for heat orchestration 505 correspondingly cab be determined based on modeling a distribution of the computational workload across the components of FIGs. 4A and 4B comprising but not limited to the central processing unit, the graphics processing unit, the artificial intelligence accelerator, an inertial measurement unit, a wireless transmitter, or a combination thereof of the wearable device.

In addition or alternatively, heat orchestration 505 can comprise delegation between a wearable device 101 and a cloud server or component. For example, delegating a computational workload from a wearable device 101 to a cloud server involves offloading tasks from the wearable device 101 to a remote server for processing. This is typically done through wireless communication, such as Wi-Fi or cellular networks, to transmit data and instructions to the cloud server. The server then executes the tasks using its resources, which are often more powerful than and not susceptible to skin contact heat compared to on-device at the wearable device 101, and returns the results back to the wearable device 101.

Example pseudo code for implementing the WATT computation 503 is provided in Tables 12-15 below. It is noted that the pseudo code is provided by way of illustration and not as limitations. It is contemplated that equivalent code or procedures can be used according to the various embodiments described herein. In one embodiment, the heat orchestration circuitry 427 or equivalent device/apparatus can execute computer code or instructions derived from the pseudo code to perform heat orchestration 505 according to various embodiments described herein.

Table 12 below illustrates pseudo code for initializing variables and data structures for heat orchestration 505, according to one example embodiment.

**Table 12**

| |
|---|
| ```
 # Initialize variables
 initialize M = {} # Initialize an empty mapping of tasks to device
``` |

Table 13 below illustrates pseudo code for performing heat orchestration 505, according to one example embodiment.

**Table 13**

| |
|---|
| ```
 # Function to perform heat orchestration
 function heat_orchestration(T, D, skin_contact_temp, WATT):
   if skin _contact_temp > WATT:
     # Skin-contact temperature exceeds WATT, orchestrate heat distribution
     for task in T:
       if task_needs_offloading(task):
         # Find the device with the highest thermal budget
         target_device = find_target_device(D)
         # Map the task to the target device
         M[task] = target_device
         # Perform device-to-device communication for workload offloading
         offload_workload(task, target_device)
``` |
| ```
   # Return the mapping of tasks to devices
   return M
``` |

The example pseudo code of Table 13 calls a heat_orchestration function that aims to prevent a skin temperature at a skin contact point of device from injuring or causing discomfort to a user. The function activates when the skin_contact_temp goes above the safe threshold (WATT). The function then analyzes a list of tasks (T) and, with the help of task_needs_offloading, identifies those that are heat-intensive. For these tasks, it searches through available devices (D) to find one with the most thermal headroom (find_target_device). Once a device with a higher thermal budget is found, the function creates a plan (M) to move tasks (e.g., ML learning or inference tasks) to this target device and then executes the actual transfer (offload_workload). Finally, it returns the updated plan (M) indicating how tasks have been redistributed for better heat management.

Table 14 below illustrates pseudo code for finding the device 101 with the highest thermal budget for heat orchestration 505, according to one example embodiment.

**Table 14**

| |
|---|
| ```
 # Function to find the device with the highest thermal budget
 function find_target_device(D):
   lowest_temp_diff = INFINITY
   target_device = None
   for device in D:
     temp_diff = skin_temp_difference(device)
     if temp_diff < lowest_temp_diff:
       lowest_temp_diff = temp_diff
       target _device = device
   return target_device
``` |

The example pseudo code of Table 14 calls a find_target_device function to search through a list of devices (D) to find the one with the greatest capacity to handle extra heat (i.e., the highest thermal budget). It does this by calculating a temp_diff (e.g., by using a function called skin_temp_difference) for each device, measuring how far below the heat threshold that device currently is. The device with the biggest temp_diff (the coolest one) is tagged as the target_device and returned as a candidate to take on some of the heat load from other devices.

Table 15 below illustrates pseudo code for the main program for heat orchestration 505, according to one example embodiment.

**Table 15**

| |
|---|
| ```
 # Main program
 T = get_tasks() # Get the set of tasks
 D = get_devices() # Get the set of devices
 skin_contact_temp = get_skin_contact_temperature() # Get the predicted skin-contact
 temperature
 WATT = get_watt_threshold() # Get the Wearability-aware temperature threshold
 # Perform heat orchestration
 heat_mapping = heat_orchestration(T, D, skin_contact_temp, WATT)
``` |

The example pseudo code of Table 15 is the main program to manage heat among a device group 113. It starts by gathering information: the tasks the devices are running (get_tasks), the available devices (get_devices), the estimated skin contact temperature (get_skin_contact_temperature), and the heat threshold (get_watt_threshold). Equipped with this knowledge, the core logic then calls the heat_orchestration function. This function analyzes the data and generates a plan (heat_mapping) detailing how to redistribute tasks across devices to prevent overheating.

In one embodiment, the multiple-device wearability-aware heat orchestration runs locally (e.g., at one of the participating devices 101) which enables privacy protection and embedded security. In addition, local analytics can be used to for modeling skin-contact temperatures and/or wearable temperature thresholds.

In other embodiments, a server-side or cloud-based trusted platform can be used in place of or in addition to one or more devices targeted for delegation of at least a portion of the computational workload. Under these cloud-based embodiments, the cloud server (not pictured) can perform one or more functions of the task orchestration manager 115 over a communications network 109 for delivery of completed computation tasks back to the wearable device 101.

Returning to FIG. 1, in one example embodiment, the devices/UEs 101 can comprise but are not limited to one or more of the form factors illustrated in FIG. 2 and may also comprise but are limited to one or more devices or apparatuses, for example, standalone cameras, IoT devices, and/or any other device capable of multiple-device wearability-aware heat orchestration according to the various example embodiments described herein. By way of example, the devices 101 (e.g., UEs) can be any type of wearables (e.g., smart glasses, augmented reality (AR) glasses, smart visor or shield), mobile terminal, user terminal, fixed terminal, or portable terminal comprising a mobile handset, station, unit, device, mobile communication device, multimedia computer, multimedia tablet, Internet node, communicator, desktop computer, laptop computer, notebook computer, netbook computer, tablet computer, personal communication system (PCS) device, personal navigation device, personal digital assistants (PDAs), point of sales (POS) device, audio/video player, digital camera/camcorder, positioning device, fitness device, television receiver, radio broadcast receiver, electronic book device, game device, display device, vehicle, smart lamppost, smart streetlight, comprising the accessories and peripherals of these devices, or any combination thereof. It is also contemplated that a device/UE 101 can support any type of interface to the user (such as "wearable" circuitry, etc.). In one instance, an IoT device may comprise one or more remote sensor devices, a wearable, a UE, or a combination thereof. Also, the devices/UEs 101 may be configured to access the communications network by way of any known or still developing communication protocols. In one example embodiment, privacy preserving masking function (e.g., encoding and/or decoding) can be implemented in any of the above-mentioned device or apparatus.

In one example, the devices/UEs 101 comprise one or more sensors comprising but not limited to cameras, LiDAR (light detection and ranging) sensor, global positioning system (GPS) sensors, sound sensors, radars, infrared (IR) light sensors, microphones, height or elevation sensors, accelerometers, tilt sensors, moisture/humidity sensors, pressure sensors, temperature sensor, barometer, NFC sensors, wireless network sensors, etc.) and clients (e.g., mapping applications, navigation applications, image processing applications, augmented reality applications, image/video application, modeling application, communication applications, etc.).

In one instance, the devices/UEs 101 and/or task orchestration manager 115 can comprise one or more neural networks or other machine learning algorithms/systems to model skin-contact temperatures and/or WATTs (e.g., on a device specific basis).

In one example, the devices/UEs 101 have communication connectivity to one or more services platforms (e.g., services platform 117) and/or one or more software applications that provides one or more services 119 that can use the functions and outputs of the system 100. By way of example, the communication connectivity can be internal connection within the apparatuses and/or happen over the communications network 109. By way of example, the one or more services 119 may also comprise mapping services, navigation services, notification services, social networking services, content (e.g., audio, video, images, etc.) provisioning services, application services, storage services, virtual reality/augmented reality (VR/AR) services, location-based services, information-based services (e.g., weather, news, etc.), payment services, market place services, data analytics services, etc. or any combination thereof.

In one example, the devices/UEs 101 and other components of the system 100 may communicate over one or more communications networks 109 that comprises one or more networks such as a data network, a wireless network, a telephony network, or any combination thereof. It is contemplated that the communications network 109 may be any local area network (LAN), metropolitan area network (MAN), wide area network (WAN), a public data network (e.g., the Internet), short range wireless communications network, or any other suitable packet-switched network, such as a commercially owned, proprietary packet-switched network, e.g., a proprietary cable or fiber-optic network, and the like, or any combination thereof. In addition, the communications network 109 may be, for example, a cellular telecom network and may employ various technologies comprising enhanced data rates for global evolution (EDGE), general packet radio service (GPRS), global system for mobile communications (GSM), Internet protocol multimedia subsystem (IMS), universal mobile telecommunications system (UMTS), worldwide interoperability for microwave access (WiMAX), Long Term Evolution (LTE) networks, 5G/3GPP (fifth-generation technology standard for broadband cellular networks / 3^{rd} Generation Partnership Project) or any further generation, code division multiple access (CDMA), wideband code division multiple access (WCDMA), wireless fidelity (Wi-Fi), wireless LAN (WLAN), Bluetooth^{®}, UWB (Ultra-wideband), Internet Protocol (IP) data casting, satellite, mobile ad-hoc network (MANET), and the like, or any combination thereof.

In one example, the system 100 or any of its components may be a platform with multiple interconnected components (e.g., a distributed framework). The system 100 and/or any of its components may comprise multiple servers, intelligent networking devices, computing devices, components, and corresponding software for spatial-temporal authentication. In addition, it is noted that the system 100 or any of its components may be a separate entity, a part of the one or more services, a part of a services platform, or included within other devices, or divided between any other components.

By way of example, the components of the system 100 can communicate with each other and other components external to the system 100 using well known, new or still developing protocols. In this context, a protocol comprises a set of rules defining how the network nodes, e.g. the components of the system 100, within the communications network interact with each other based on information sent over the communication links. The protocols are effective at different layers of operation within each node, from generating and receiving physical signals of various types, to selecting a link for transferring those signals, to the format of information indicated by those signals, to identifying which software application executing on a computer system sends or receives the information. The conceptually different layers of protocols for exchanging information over a network are described in the Open Systems Interconnection (OSI) Reference Model.

Communications between the network nodes are affected by exchanging discrete packets of data. The packets may comprise, for example, (1) header information associated with a particular protocol, and (2) payload information that follows the header information and contains information that may be processed independently of that particular protocol. In some protocols, the packet comprises (3) trailer information following the payload and indicating the end of the payload information. The header comprises information such as the source of the packet, its destination, the length of the payload, and other properties used by the protocol. Often, the data in the payload for the particular protocol comprises a header and payload for a different protocol associated with a different, higher layer of the OSI Reference Model. The header for a particular protocol, for example, indicates a type for the next protocol contained in its payload. The higher layer protocol is said to be encapsulated in the lower layer protocol. The headers included in a packet traversing multiple heterogeneous networks, such as the Internet, typically comprise a physical (layer 1) header, a data-link (layer 2) header, an internetwork (layer 3) header and a transport (layer 4) header, and various application (layer 5, layer 6 and layer 7) headers as defined by the OSI Reference Model.

The processes described herein for providing multiple-device wearability-aware heat orchestration may be advantageously implemented via software, hardware (e.g., general processor, Digital Signal Processing (DSP) chip, an Application Specific Integrated Circuit (ASIC), Field Programmable Gate Arrays (FPGAs), etc.), firmware, circuitry, or a combination thereof. Such exemplary hardware for performing the described functions is detailed below.

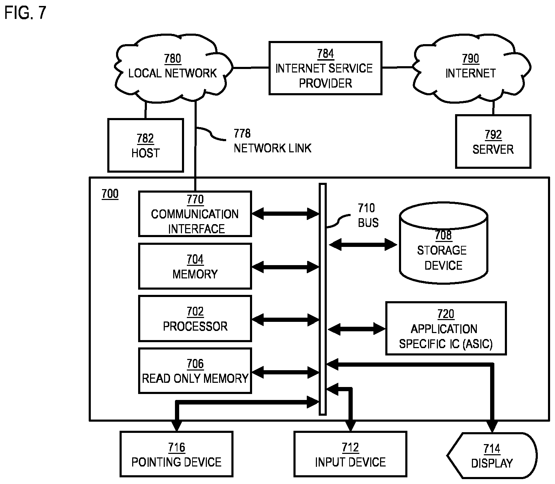

FIG. 7 illustrates an example computer system 700 upon which embodiments of the invention as described with the processes described herein may be implemented. The computer system 700 is programmed (e.g., via computer program code or instructions) to provide multiple-device wearability-aware heat orchestration as described herein and comprises a communication mechanism such as a bus 710 for passing information between other internal and external components of the computer system 700. Information (also called data) is represented as a physical expression of a measurable phenomenon, e.g. electric voltages, but comprising, in other embodiments, such phenomena as magnetic, electromagnetic, pressure, chemical, biological, molecular, atomic, sub-atomic and quantum interactions. For example, north and south magnetic fields, or a zero and non-zero electric voltage, represent two states (0, 1) of a binary digit (bit). Other phenomena can represent digits of a higher base. A superposition of multiple simultaneous quantum states before measurement represents a quantum bit (qubit). A sequence of one or more digits constitutes digital data that is used to represent a number or code for a character. In some embodiments, information called analog data is represented by a near continuum of measurable values within a particular range.

A bus 710 comprises one or more parallel conductors of information so that information is transferred quickly among devices coupled to the bus 710. One or more processors 702 for processing information are coupled with the bus 710.

A processor 702 performs a set of operations on information as specified by computer program code related to providing multiple-device wearability-aware heat orchestration. The computer program code is a set of instructions or statements providing instructions for the operation of the processor and/or the computer system to perform specified functions. The code, for example, may be written in a computer programming language that is compiled into a native instruction set of the processor. The code may also be written directly using the native instruction set (e.g., machine language). The set of operations comprises bringing information in from the bus 710 and placing information on the bus 710. The set of operations may also comprise comparing two or more units of information, shifting positions of units of information, and combining two or more units of information, such as by addition or multiplication or logical operations like OR, exclusive OR (XOR), and AND. Each operation of the set of operations that can be performed by the processor is represented to the processor by information called instructions, such as an operation code of one or more digits. A sequence of operations to be executed by the processor 702, such as a sequence of operation codes, constitute processor instructions, also called computer system instructions or, simply, computer instructions. Processors may be implemented as mechanical, electrical, magnetic, optical, chemical or quantum components, among others, alone or in combination.

The computer system 700 also comprises a memory 704 coupled to bus 710. The memory 704, such as a random access memory (RAM) or other dynamic storage device, stores information comprising processor instructions for providing multiple-device wearability-aware heat orchestration. Dynamic memory allows information stored therein to be changed by the computer system 700. RAM allows a unit of information stored at a location called a memory address to be stored and retrieved independently of information at neighboring addresses. The memory 704 is also used by the processor 702 to store temporary values during execution of processor instructions. The computer system 700 also comprises a read only memory (ROM) 706 or other static storage device coupled to the bus 710 for storing static information, comprising instructions, that is not changed by the computer system 700. Some memory is composed of volatile storage that loses the information stored thereon when power is lost. Also coupled to bus 710 is a non-volatile (persistent) storage device 708, such as a magnetic disk, optical disk or flash card, for storing information, comprising instructions, that persists even when the computer system 700 is turned off or otherwise loses power.

Information, comprising instructions for providing multiple-device wearability-aware heat orchestration, is provided to the bus 710 for use by the processor from an external input device 712, such as a keyboard containing alphanumeric keys operated by a human user, or one or more sensors. A sensor detects conditions in its vicinity and transforms those detections into physical expression compatible with the measurable phenomenon used to represent information in the computer system 700. Other external devices coupled to bus 710, used primarily for interacting with humans, comprise a display device 714, such as a cathode ray tube (CRT) or a liquid crystal display (LCD), or plasma screen or printer for presenting text or images, and a pointing device 716, such as a mouse or a trackball or cursor direction keys, or motion sensor, for controlling a position of a small cursor image presented on the display 714 and issuing commands associated with graphical elements presented on the display 714. In some embodiments, for example, in embodiments in which the computer system 700 performs all functions automatically without human input, one or more of external input device 712, display device 714 and pointing device 716 is omitted. In various embodiment, the computer system 700 is further connected via the bus 710 to a one or more camera device, flash device or Lidar device.

In the illustrated example embodiment, special purpose hardware, such as an application specific integrated circuit (ASIC) 720, is coupled to bus 710. The special purpose hardware is configured to perform operations not performed by processor 702 quickly enough for special purposes. Examples of application specific ICs comprise graphics accelerator cards for generating images for display 714, cryptographic boards for encrypting and decrypting messages sent over a network, speech recognition, and interfaces to special external devices, such as robotic arms and medical scanning equipment that repeatedly perform some complex sequence of operations that are more efficiently implemented in hardware.

Computer system 700 also comprises one or more instances of a communications interface 770 coupled to bus 710. Communication interface 770 provides a one-way or two-way communication coupling to a variety of external devices that operate with their own processors, such as printers, scanners and external disks. In general, the coupling is with a network link 778 that is connected to a local network 780 to which a variety of external devices with their own processors are connected. For example, communication interface 770 may be a parallel port or a serial port or a universal serial bus (USB) port on a personal computer. In some embodiments, communications interface 770 is an integrated services digital network (ISDN) card or a digital subscriber line (DSL) card or a telephone modem that provides an information communication connection to a corresponding type of telephone line. In some embodiments, a communication interface 770 is a cable modem that converts signals on bus 710 into signals for a communication connection over a coaxial cable or into optical signals for a communication connection over a fiber optic cable. As another example, communications interface 770 may be a local area network (LAN) card to provide a data communication connection to a compatible LAN, such as Ethernet. Wireless links may also be implemented. For wireless links, the communications interface 770 sends or receives or both sends and receives electrical, acoustic or electromagnetic signals, comprising infrared and optical signals, that carry information streams, such as digital data. For example, in wireless handheld devices, such as mobile telephones like cell phones, the communications interface 770 comprises a radio band electromagnetic transmitter and receiver called a radio transceiver. In certain embodiments, the communications interface 770 enables connection to the communications network 109 for providing multiple-device wearability-aware heat orchestration.

The term computer-readable medium is used herein to refer to any medium that participates in providing information to processor 702, comprising instructions for execution. Such a medium may take many forms, comprising, but not limited to, non-volatile media, volatile media and transmission media. Non-volatile media comprise, for example, optical or magnetic disks, such as storage device 708. Volatile media comprise, for example, dynamic memory 704. Transmission media comprise, for example, coaxial cables, copper wire, fiber optic cables, and carrier waves that travel through space without wires or cables, such as acoustic waves and electromagnetic waves, comprising radio, optical and infrared waves. Signals comprise man-made transient variations in amplitude, frequency, phase, polarization or other physical properties transmitted through the transmission media. Common forms of computer-readable media comprise, for example, a floppy disk, a flexible disk, hard disk, magnetic tape, any other magnetic medium, a CD-ROM, CDRW, DVD, any other optical medium, punch cards, paper tape, optical mark sheets, any other physical medium with patterns of holes or other optically recognizable indicia, a RAM, a PROM, an EPROM, a FLASH-EPROM, any other memory chip or cartridge, a carrier wave, or any other medium from which a computer can read.

Network link 778 typically provides information communication using transmission media through one or more networks to other devices that use or process the information. For example, network link 778 may provide a connection through local network 780 to a host computer 782 or to equipment 784 operated by an Internet Service Provider (ISP). ISP equipment 784 in turn provides data communication services through the public, world-wide packet-switching communications network of networks now commonly referred to as the Internet 790.

A computer called a server host 792 connected to the Internet hosts a process that provides a service in response to information received over the Internet. For example, server host 792 hosts a process that provides information representing video data for presentation at display 714. It is contemplated that the components of the system 100 can be deployed in various configurations within other computer systems, e.g., host 782 and server 792.

FIG. 8 illustrates a chip set 800 upon which embodiments of the invention, for example, the components (e.g., devices/UEs 101, task orchestration manager 115, a services platform 117, services 119, and content providers 121) may be implemented. The chip set 800 is programmed to provide multiple-device wearability-aware heat orchestration as described herein and comprises, for instance, the processor and memory components described with respect to FIG. 5 incorporated in one or more physical packages (e.g., chips). By way of example, a physical package comprises an arrangement of one or more materials, components, and/or wires on a structural assembly (e.g., a baseboard) to provide one or more characteristics such as physical strength, conservation of size, and/or limitation of electrical interaction. It is contemplated that in certain embodiments the chip set can be implemented in a single chip.

In one embodiment, the chip set 800 comprises a communication mechanism such as a bus 801 for passing information among the components of the chip set 800. A processor 803 has connectivity to the bus 801 to execute instructions and process information stored in, for example, a memory 805. The processor 803 may comprise one or more processing cores with each core configured to perform independently. A multi-core processor enables multiprocessing within a single physical package. Examples of a multi-core processor comprise two, four, eight, or greater numbers of processing cores. Alternatively or in addition, the processor 803 may comprise one or more microprocessors configured in tandem via the bus 801 to enable independent execution of instructions, pipelining, and multithreading. The processor 803 may also be accompanied with one or more specialized components to perform certain processing functions and tasks such as one or more digital signal processors (DSP) 807, or one or more application-specific integrated circuits (ASIC) 809. A DSP 807 typically is configured to process real-world signals (e.g., sound) in real time independently of the processor 803. Similarly, an ASIC 809 can be configured to perform specialized functions not easily performed by a general purposed processor. Other specialized components to aid in performing the inventive functions described herein comprise one or more field programmable gate arrays (FPGA) (not shown), one or more controllers (not shown), or one or more other special-purpose computer chips.

The processor 803 and accompanying components have connectivity to the memory 805 via the bus 801. The memory 805 comprises both dynamic memory (e.g., RAM, magnetic disk, writable optical disk, etc.) and static memory (e.g., ROM, CD-ROM, etc.) for storing executable instructions that when executed perform the inventive steps described herein to provide multiple-device wearability-aware heat orchestration. The memory 805 also stores the data associated with or generated by the execution of the inventive steps.

## Claims

1. An apparatus comprising:
means for determining a predicted skin temperature at a skin contact point of a wearable device;
means for determining a wear status of the wearable device;
means for determining a wearable temperature threshold of the wearable device based on the wear status; and
means for delegating at least one portion of a computational workload of the wearable device to at least one other device based on determining that the predicted skin temperature is greater than the wearable temperature threshold.

2. The apparatus according to claim 1, wherein the apparatus further comprises:
means for modeling a relationship between the computational workload of the wearable device and a skin temperature measured at the skin contact point,
wherein the predicted skin temperature at the skin contact point is based on the modeling.

3. The apparatus according to any of the preceding claims, wherein the computational workload comprises a machine learning inference task.

4. The apparatus according to any of the preceding claims, wherein the apparatus further comprises:
means for determining a measured skin temperature at the skin contact point,
wherein the predicted skin temperature is further based on the measured skin temperature.

5. The apparatus according to any of the preceding claims, wherein the apparatus further comprises:
means for determining contextual data associated with the wearable device, a user of the wearable device, an environment in which the wearable device is operating, or a combination thereof,
wherein the predicted skin temperature is further based on the contextual data.

6. The apparatus according to any of the preceding claims, wherein the apparatus further comprises:
means for determining a user's perceived temperature comfort level,
wherein the wearable temperature threshold is further based, at least in part, on the user's perceived temperature comfort level.

7. The apparatus according to any of the preceding claims, wherein the wear status comprises an on-body status that indicates that the wearable device is being worn, and an off-body status that indicates that the wearable device is not being worn.

8. The apparatus according to claim 7, wherein the wearable temperature threshold is set to a maximum temperature at which the wearable device can operate without reducing a quality of service based on determining that the wear status is the off-body status.

9. The apparatus according to any of the preceding claims, wherein the apparatus further comprises:
means for determining another wearable temperature threshold for the at least one other device,
wherein the delegating of the least one portion of the computational workload to the at least one other device is further based, at least in part, on the another wearable temperature threshold.

10. The apparatus according to any of claims 1-8, wherein the apparatus further comprises:
means for determining one or more respective wearable temperature thresholds for one or more candidate devices; and
means for selecting the at least one other device to which the at least one portion of the computational workload is delegated based on the one or more respective wearable temperature thresholds.

11. The apparatus according to any of the preceding claims, wherein the delegating of the at least one portion of the computational workload is performed using a device-to-device communication.

12. The apparatus according to any of the preceding claims, wherein the computational workload is distributed across at least one of a central processing unit, a graphics processing unit, an artificial intelligence accelerator, an inertial measurement unit, a wireless transmitter, or a combination thereof of the wearable device.

13. The apparatus according to any of claims 1-12, wherein the predicted skin temperature is determined based on modeling a distribution of the computational workload across a central processing unit, a graphics processing unit, an artificial intelligence accelerator, an inertial measurement unit, a wireless transmitter, or any combination thereof of the wearable device.

14. A method comprising:
determining a predicted skin temperature at a skin contact point of a wearable device;
determining a wear status of the wearable device;
determining a wearable temperature threshold of the wearable device based on the wear status; and
delegating at least one portion of a computational workload of the wearable device to at least one other device based on determining that the predicted skin temperature is greater than the wearable temperature threshold.

15. A computer program comprising instructions, when executed by an apparatus, cause the apparatus to perform at least the following:
determining a predicted skin temperature at a skin contact point of a wearable device;
determining a wear status of the wearable device;
determining a wearable temperature threshold of the wearable device based on the wear status; and
delegating at least one portion of a computational workload of the wearable device to at least one other device based on determining that the predicted skin temperature is greater than the wearable temperature threshold.
